# EUROPÄISCHE PATENTSCHRIFT

(11) **EP 1 509 512 B1**
(45) Veröffentlichungstag und Bekanntmachung des Hinweises auf die Patenterteilung: **14.02.2007**
(21) Anmeldenummer: 03752664.7
(22) Anmeldetag: 13.05.2003
(51) Int. Cl.: C07D 323/06

(54) **VERFAHREN ZUR HERSTELLUNG VON REINEM TRIOXAN**
METHOD FOR PRODUCING PURE TRIOXANE
PROCEDE DE FABRICATION DE TRIOXANE PUR

(30) Priorität: 17.05.2002 DE 10222163
(43) Veröffentlichungstag der Anmeldung: 02.03.2005
(73) Patentinhaber: BASF Aktiengesellschaft, 67056 Ludwigshafen (DE)
(72) Erfinder: FRIESE, Katrin, 68163 Mannheim (DE); RAULS, Matthias, 67061 Ludwigshafen (DE); FREYHOF, Reinhard, 67227 Frankenthal (DE); FRIESE, Thorsten, 68163 Mannheim (DE); ARMBRUSTER, Harald, 67117 Limburgerhof (DE); ZEINER, Hartmut, 67071 Ludwigshafen (DE); EGBERS, Gitta, 49080 Osnabrück (DE); STRÖFER, Eckhard, 68163 Mannheim (DE); HECK, Ludwig, 68535 Edingen-Neckarhausen (DE); HILDENBRAND, Peter, 76199 Karlsruhe (DE)
(86) Internationale Anmeldenummer: PCT/EP2003/004968
(87) Internationale Veröffentlichungsnummer: WO 2003/097630

(56) Entgegenhaltungen:
- EP-A- 0 036 552
- EP-A- 0 573 850

## Beschreibung

Die Erfindung betrifft ein verbessertes Verfahren zur Herstellung von reinem Trioxan.

Bei der Herstellung von Trioxan (s. beispielsweise DE-A 1543390) entsteht ein Gemisch, das im wesentlichen aus Trioxan, Wasser und Formaldehyd besteht. Aus diesem Gemisch wird Trioxan extraktiv mit Hilfe eines Schleppmittels, wie z.B. chlorhaltige Schleppmittel wie Methylenchlorid oder Benzol abgetrennt. Weitere Bestandteile des Gemisches in untergeordneten Mengen sind in der Regel Ameisensäure, Methylal und Dimethoxydimethylether sowie Methanol und Methylformiat. In einer nachfolgenden Destillation wird das Schleppmittel zurückgewonnen und der Extraktion wieder zugeführt. Bei diesem Verfahren (siehe z.B. EP-A 583 907) müssen große Mengen Schleppmittel eingesetzt und mit hohem Energieaufwand zurückgewonnen werden. Zwangsweise anfallende Emissionen müssen aufwendig entsorgt werden, da Methylenchlorid und Benzol als gefährliche Schadstoffe eingestuft sind.

EP-A-36552 offenbart ein Verfahren zur Reinigung von Trioxan durch Behandlung des Rohtrioxans in der Schmelze mit Kalium- oder Bariumhydroxid.

Das Extrakt wird neutralisiert; die hierbei entstehenden Salze werden ausgewaschen, da sie schon in geringen Konzentrationen die Polymerisation von Trioxan beeinflussen. In einer anschließenden Reindestillation des Extraktes werden das Extraktionsmittel und störende Nebenkomponenten abgetrennt, um ein polymerisationsfähiges Trioxan zu erhalten. Für die Aufarbeitung des wässrigen formaldehydhaltigen Raffinates, des Extraktionsmittels sowie der salzhaltigen Ablauge sind weitere Destillationsschritte erforderlich.

Die Extraktion stellt in diesem Verfahren einen besonders aufwendigen Schritt zur Reinigung von Trioxan dar und ist zudem mit dem Einsatz von aus ökologischer Sicht unerwünschten Chlorverbindungen, wie z.B. Methylenchlorid, verbunden. Hiermit sind strenge Umweltauflagen verbunden. Bei der Extraktion bleiben - je nach Extraktionsmittel - zum Teil erhebliche Mengen Trioxan in der wässrigen Phase. Hierdurch ergeben sich große Kreislaufströme. Mit dem organischen Lösungsmittel wird ein zusätzlicher Stoff in das Verfahren eingebracht. Hierdurch wird eine nachfolgende Aufarbeitung der organischen. Phase erforderlich, die mit Verlusten von Trioxan verbunden ist. Die in der Extraktionsstufe enthaltenen Waschschritte benötigen darüber hinaus allein 25 % des gesamten im Verfahren eingesetzten VE-Wassers, so dass sich mit einem Verzicht auf die Extraktion auch die aufzuarbeitenden Abwasserströme reduzieren ließen.

Aus der DE-A 350 86 68 (Hoechst AG, Frankfurt) ist die Herstellung hochreinen Trioxans aus einem wässrigen Formaldehyd/Trioxan-Gemisch durch mehrstufige Kristallisation bekannt, die zumindest teilweise als Schmelzekristallisation (Schichtkristallisation) ausgeführt wird. Eingesetzt wird hier entweder direkt das bei der Trioxan-Synthese anfallende wässrige Gemisch oder ein bereits vorgereinigtes, weitgehend wasserfreies Gemisch. Nach der Lehre der Schrift ergeben sich sinnvolle Trioxan-Ausbeuten aufgrund der Lage des Eütektikums nur bei Trioxan-Konzentrationen im Ausgangsgemisch von mindestens 50 Gew.-%, bevorzugt mindestens 95 Gew.-%. Bei einem hohen Wassergehalt des Ausgangsgemisches (30 bis 50 Gew. -%) wird die erste Stufe der Kristallisation als Lösungskristallisation ausgeführt. Um eine Trioxan-Konzentration von mindestens 50 Gew.-% im Ausgangsgemisch zu erzeugen, sind nach der Synthese weitere Aufarbeitungsschritte erforderlich. Schichtkristallisationsverfahren werden häufig bei höheren Ausgangskonzentrationen des Wertproduktes (> 95 Gew.-%) eingesetzt. Um eine Trioxankonzentration von 95 Gew.-% im Ausgangsgemisch einzustellen, muss das Azeotrop Trioxan/Wasser übersprungen werden. Damit geht eine wichtige Zielsetzung der Kristallisation, nämlich die Einsparung von Verfahrensschritten durch direkte Kristallisation nach dem Syntheseschritt, verbunden mit einem Überspringen des Azeotrops, verloren. Zudem sind weitere nachfolgende Behandlungen des Trioxans auch bei höherer Reinheit erforderlich, um die erforderlichen geringen Wassergehalte (< 50 ppm) für die anschließende Polymerisation zu erzielen.

Die Trennung von Trioxan aus gasförmigen Gemischen mit Formaldehyd durch Absorption in alkoholhaltigen Flüssigkeiten und anschließender Kristallisation ist beispielsweise in den DE-A 19833620 und EP-A 976743 beschrieben. Gasförmiges Formaldehyd und Trioxan werden dabei vorzugsweise in einem ein- oder mehrwertigen Alkohol gelöst. Trioxan wird aus der Lösung entweder in einer Schichtkristallisation oder in einem kontinuierlichen Suspensionsverfahren auskristallisiert und danach abgetrennt.

Bei einem Schichtkristallisationsverfahren wird an gekühlten Flächen Kristallisat in Form zusammenhängender, fest anhaftender Schichten ausgefroren. Die Fest/Flüssig-Trennung erfolgt durch einfaches Ablassen der Restschmelze; das gereinigte Kristallisat wird aufgeschmolzen. Hohe Reinheiten und Ausbeuten werden in der Regel erst durch wiederholtes, taktweises Kristallisieren erreicht. In jeder Stufe muss dabei die Kristallisationswärme beim Ausfrieren ab- und beim anschließenden Aufschmelzen wieder zugeführt werden. Zusätzlich muss auch der Apparat selbst jeweils aufgeheizt und wieder abgekühlt werden. Die Trioxanausbeute bei. den oben genannten Verfahren beträgt lediglich ca. 51,4 %.

Alternativ zur Schichtkristallisation wird in der EP-A 976 743 ein Verfahren zur Suspensionskristallisation von Trioxan beschrieben.

Bei dem im Stand der Technik beschriebenen Kristallisations-Verfahren wird gasförmiges Trioxan verarbeitet. Der Kristallisation vorgeschaltet ist zwangsläufig die Absorption des Trioxans in einer geeigneten Lösung. Zusätzliche Verfahrensschritte und Lösemittel sind jedoch -wie bereits erläutert- nicht prozeßökonomisch und somit nachteilig bei dem beschriebenen Verfahren.

Aus der EP-A 573 850 geht die Herstellung von polymerisationsfähigem Trioxan hoher Reinheit durch mehrstufige Kristallisation mit Additiven hervor. Durch Zugabe von Additiven (alkalische organische Verbindungen,' z.B. tertiäre Amine) wird die Bildung von "Flusen", die nach mehreren Betriebsstunden bzw. Kristallisationszyklen bei hohen Trioxangehalten (> 95 Gew.-%) vermutlich durch Paraformaldehyd-Bildung entstehen können, wirksam verhindert. Die Additive werden beim Schwitzen und/oder Waschen so weit entfernt, daß sie analytisch nicht mehr nachweisbar sind und die Polymerisation des Rein-Trioxans nicht beeinträchtigen. Das für die Kristallisation eingesetzte Gemisch weist hier bereits einen Trioxangehalt von 94 Gew.-% auf. Eine zweistufige Schichtkristallisation liefert Trioxan mit einer Reinheit von 99,9 Gew.-%. Die hier beschriebene Schichtkristallisation weist die bereits oben genannten Nachteile eines hohen Energieaufwandes und der Notwendigkeit einer hohen Ausgangskonzentration an Trioxan auf.

Gemäß der EP-A 248 487 kann Polyoxymethylen aus Trioxan hergestellt werden, das nach der Synthese durch Rektifikation aufkonzentriert und dann durch Extraktion mit aliphatischen Kohlenwasserstoffen aus dem wässrigen Synthesegemisch abgetrennt' wird. Der Extrakt (Trioxan in organischem Exktraktionslösemittel) wird anschließend kristallisiert. Das durch Kristallisation gereinigte Trioxan wird resuspendiert und aus der Suspension heraus polymerisiert. Die Nachteile einer extraktiven Abtrennung wurden bereits oben beschrieben.

In DE 19 842 579 wird ein Verfahren zur Abtrennung von Trioxan aus flüssigen Gemischen beschrieben, bei dem das Trioxan durch Verdunsten oder Verdampfen möglichst selektiv in die Gasphase überführt und anschließend durch Abkühlung und Kondensation oder Desublimation flüssig oder fest gewonnen wird. Problematisch bei einer Verdampfung des Trioxans bei Normaldruck ist die hohe thermische Belastung. Sinnvoll aber unökonomisch ist daher der Einsatz eines Trägergases, mit dem das Trioxan aus dem flüssigen Gemisch ausgestrippt wird. Das beschriebene Verfahren lässt für die Aufarbeitung einer wasserhaltigen Trioxan-Lösung somit keine Vorteile erkennen, zumal die Trioxan-Konzentration schon vor den beiden Reinigungsschritten Verdampfung und Desublimation jenseits des Azeotrops von Trioxan und Wasser liegt und zusätzlich weitere Verfahrensschritte zur Erzeugung reinen Trioxans erforderlich sind.

Aus der US 2,465,489 ist ein Verfahren zur Gewinnung von Trioxan aus wässrigen Lösungen durch Kristallisation bekannt. Die Abtrennung von Resten des Formaldehyds erfolgt durch Waschen mit Methanol/Ethanol. Hierbei gehen jedoch ca. 30 % des Trioxans ebenso in Lösung, wodurch sich die Trioxanausbeute entsprechend reduziert. Nachteilig ist weiterhin auch die hohe Ausgangskonzentration von mind. ca. 50 Gew.-% an Trioxan im Gemisch, die nur durch zusätzliche vorgeschaltete Verfahrensschritte erzielt werden kann.

Aufgabe der vorliegenden Erfindung war es daher, ein Verfahren zur Herstellung und anschließender Abtrennung des Trioxans bereit zu stellen, welches eine möglichst vollständige Gewinnung des Trioxans bietet und möglichst wenig zusätzliche Lösemittel oder Verfahrensschritte erfordert sowie weniger Nebenprodukte entstehen lässt. Das erhaltene Trioxan sollte möglichst rein sein bzw. in hohen Ausbeuten erhältlich sein, wobei insbesondere der Wassergehalt möglichst gering sein soll (unterhalb 50 ppm), da die Polymerisationsfähigkeit ansonsten nicht gegeben ist.

Demgemäß wurde ein Verfahren zur Herstellung von Trioxan durch Trimerisierung von Formaldehyd in wässriger saurer Lösung und anschließender Abtrennung von Trioxan aus einem im wesentlichen aus Trioxan, Wasser und Formaldehyd bestehenden Gemisch (Trioxan-Rohprodukt) gefunden, welches dadurch gekennzeichnet ist, dass man
a) das Trioxan-Rohprodukt aus der Trimerisierung destilliert,
b) das Trioxan auskristallisiert und
c) von der Mutterlauge abtrennt sowie
d) anschließend aufschmilzt und
e) weitere Nebenprodukte abdestilliert.

Die Herstellung von Trioxan aus Formaldehyd (Trimerisierung) in Gegenwart von wässrigen sauren Lösungen ist dem Fachmann bekannt, weshalb sich weitere Angaben hierzu erübrigen.

Das Trioxan-Rohprodukt (Gemisch nach der Trimerisierung) enthält erfindungsgemäß Trioxangehalte von 30 bis 55 Gew.-%, vorzugsweise von 35 bis 46 Gew.-% bei einem Formaldehydanteil von 15 bis 30 Gew.-%, vorzugsweise 18 bis 25 Gew.-% und einem Wasseranteil von 25 bis 40 Gew.-%, vorzugsweise von 28 bis 38 Gew.-%.

Als Nebenprodukte aus der Trimerisierung sind in der Regel Methanol (< 5 Gew.-%), Methylformiat (< 5 Gew.-%), Methylal (< 4 Gew.-%), Ameisensäure (< 3 Gew.-%), Tetroxan (< 1 Gew.-%) und Dimethoxydimethylether (= DOE < 1 Gew.-%) im Trioxan-Rohprodukt vorhanden sowie auch höher siedende Nebenkomponenten.

Gemäß der erfindungsgemäßen Abtrennungs-Verfahrensweise wird zunächst in einer Stufe a) das Trioxan-Rohprodukt aus der Trimerisierung destilliert und die Leichtsieder wie Methylformiat, Methylal, Methanol, DOE sowie auch geringe Mengen an Wasser und Formaldehyd abgetrennt.

Die Destillation kann in einer oder in mehreren Kolonnen stattfinden. Die Kopftemperatur beträgt in der Regel > 40°C, vorzugsweise > 60 °C. Vorzugsweise wird die Destillation bei Normaldruck oder bei leichtem Überdruck bis zu 2,5 bar durchgeführt.

Das Sumpfprodukt enthält überwiegend nach der Stufe a) 30 bis 55 Gew.-%, vorzugsweise 36 bis 47 Gew.-% Trioxan, 15 bis 30 Gew.-%, vorzugsweise 18 bis 25 Gew.-% Formaldehyd und 25 bis 40 Gew.-%, vorzugsweise von 28 bis 38 Gew.-% Wasser sowie geringe Anteile der bereits erwähnten Nebenprodukte.

Dieses Gemisch (Sumpfprodukt) wird erfindungsgemäß aus der Kolonne (bzw. den Kolonnen) ausgetragen und in geeignete Vorrichtungen für die Kristallisationsstufe b) überführt. Geeignete Vorrichtungen sind beispielsweise Kühlscheibenkristallisator, Rohrkristallisator oder Kratzkühler.

Die Kristallisation b) kann sowohl diskontinuierlich als auch kontinuierlich oder halb-kontinuierlich, einstufig oder mehrstufig durchgeführt werden. Als bevorzugte Verfahrensweise sei die kontinuierliche Kristallisation genannt, welche insbesondere einstufig durchgeführt wird. Die Stufe b) kann als Schmelze- und/ oder Lösungskristallisation erfolgen.

Als eine besonders bevorzugte Ausführungsform der Kristallisationsstufe b) sei beim erfindungsgemäßes Abtrennungsverfahren die Suspensionskristallisation genannt.

Die Temperaturen der Kristallisationsstufe b) des erfindungsgemäßen Verfahrens betragen von -10°C bis +65°C, vorzugsweise von 0 bis 40°C. Bevorzugte Abkühlraten bei der diskontinuierlichen Fahrweise betragen maximal 15 K/Stunde, insbesondere maximal 10 K/Stunde. Bei der kontinuierlichen Fahrweise stellt man vorzugsweise einen Temperaturgradienten entlang des Kristallisators ein. Insbesondere bevorzugt sind bei den Verfahrensweisen Endtemperaturen von ≤ 20°C, vorzugsweise ≤ 15°C. In der Regel wird Stufe b) vorteilhaft bei Normaldruck durchgeführt, es kann aber auch unter Überdruck bis zu 2,5 bar oder Unterdruck bis zu 0,3 bar gearbeitet werden, wobei verfahrenstechnische Vorteile hierbei nicht zu erwarten sind.

Nach der Stufe b) des erfindungsgemäßen Verfahrens wird die Mutterlauge vom Kristallisat abgetrennt (Stufe c) und diese gegebenenfalls vorteilhafterweise destillativ aufgearbeitet zur Rückgewinnung von Formaldehyd und Trioxan. Der überwiegend formaldehydhaltige Strom aus der Destillation der Mutterlauge wird in die Trimerisierung, der überwiegend trioxanhaltige Strom in die Leichtsiederabtrennung a) zurückgeführt.

Geeignete Vorrichtungen für die Kristallisat-Abtrennung sind jegliche Arten von Zentrifugen z.B. Schälzentrifuge und besonders Schubzentrifuge, Bandfilter oder besonders bevorzugt Waschkolonnen. Diese sind deshalb vorteilhaft, da man Stufe c) und auch die folgende Stufe d) in einer Vorrichtung durchführen kann sowie mittels einer Gegenstromwäsche das Kristallisat gegebenenfalls waschen kann.

Die Mutterlauge enthält in der Regel nach der Kristallisation maximal bis zu 20 Gew.-% Trioxan, 30 bis 40 Gew.-% Formaldehyd und 35 bis 55 Gew.-% Wasser sowie geringe Anteile an Methanol, Ameisensäure, Methylformiat, Methylal, Butandiolformal, Tetroxan.

Nach der Kristallisation b) enthält das abgetrennte Kristallisat bereits > 92 Gew.-% Trioxan, 1 bis 5 Gew.-% Formaldehyd, 1 bis 7 Gew.-% Wasser sowie < 0,3 Gew.-% Schwersieder wie beispielsweise Tetroxan, TOE = Trimethoxydimethylether.

Das Kristallisat kann gegebenenfalls nach der Abtrennung c) gewaschen werden. Geeignete Waschmittel sind Wasser und/oder Alkohole wie Methanol, Ethanol, 1 oder 2-Propanol. Vorzugsweise führt man die Waschung des Kristallisates bei Waschmittel-Temperaturen ≤ 20°C, vorzugsweise ≤ 5°C durch. Das volumetrische

Verhältnis von Kristallisat zu Waschflüssigkeit beträgt in der Regel 1:0,5 bis 1:3, vorzugsweise 1:1.

Durch das Waschen mit Wasser erhöht sich der Anteil an Wasser geringfügig, während der Formaldehyd-Anteil in der Regel unter 0,5 Gew.-% sinkt. Der Gehalt an Trioxan bleibt weitestgehend konstant > 92 Gew.-%.

Bei einer besonders bevorzugten Abfolge der Wäsche des Kristallisates wird erst mit Wasser und anschließend mit Alkoholen, vorzugsweise Methanol, gewaschen.

Das auf diese Weise behandelte Kristallisat enthält mindestens 98 Gew.-% Trioxan, weniger als 0,1 Gew.-% Formaldehyd sowie < 0,5 Gew.-% Wasser.

Das Kristallisat wird in der anschließenden Stufe d) des erfindungsgemäßen Verfahrens aufgeschmolzen.

Als geeignete Apparate für Stufe d) seien isolierte Behälter mit Aufschmelzkreislauf (Wärmetauscher mit Dampf oder Kondensat), der Sumpfkreislauf einer Waschkolonne oder Sublimatoren genannt.

Erfindungsgemäß wird das aufgeschmolzene Kristallisat in eine oder mehrere Kolonnen eingebracht und erneut destilliert (Stufe e).

Durch die Stufe e) werden insbesondere TOE, DOE, Tetroxan und Wasser abgetrennt.

Die Destillation Stufe e) wird bei einem Druck von 0,3 bis 2 bar, bevorzugt bei Normaldruck betrieben. Die Temperatur des flüssigen Zulaufs liegt unter Normaldruck bei 62°C bis 120°C. Es ist jedoch auch möglich, die Kolonne mit einem ganz oder teilweise gasförmigen Zulauf (Zulauftemperatur ≥ 120°C bei Normaldruck) zu betreiben.

Bei einer Niederdruckkolonne beträgt der Druck in der Regel bis zu 0,3 bar. Bei Temperaturen von 62 bis 80°C wird der Zulauf flüssig, bei Temperaturen > 80°C zumindest teilweise gasförmig eingesetzt.

Die wässrige Phase (Kopfprodukt) der Reindestillation (Stufe e) kann bei dem erfindungsgemäßen Verfahren vor die Stufe a) zurückgeführt werden, die Schwersieder aus der Stufe e) können in die Trimerisierung zweckmäßigerweise zurückgeführt werden.

Im Anschluß an die Reindestillation e) wird das Trioxan aus der Stufe e) ausgetragen und in den Polymerisationsprozeß überführt.

Bei dem erfindungsgemäßen Verfahren sind keine zusätzlichen Komponenten erforderlich. Die Abtrennung erfolgt in weniger Schritten und weitestgehend ohne Bildung weiterer Nebenprodukte. Darüberhinaus entsteht ein Produkt hoher Reinheit, welches nach der erfindungsgemäßen Fahrweise aus mindestens 99,5 Gew.-% Trioxan besteht. Der für die Polymerisation ausschlaggebende Anteil an Wasser beträgt < 50 ppm, der Gehalt an Ameisensäure < 5 ppm sowie der Gehalt an DOE < 100 ppm, sowie an TOE von 100 bis 200 ppm.

### Beispiele

### Beispiel 1:

### Trimerisierung von Formaldehyd, Leichtsiederabtrennung, diskontinuierliche Kristallisation von Trioxan ohne Waschschritt (Endtemperatur: 21°C), Reindestillation

Wässrige Formaldehyd-Lösung (49 Gew.-% Formaldehyd) wurde in einem Fallfilm-Verdampfer bei 68°C und 0,25 bar aufkonzentriert. Die aufkonzentrierte Formaldehyd-Lösung (63 Gew.-% Formaldehyd, 34 Gew.-% Wasser, Rest: hauptsächlich Methanol) wurde in den Sumpf der Synthesekolonne bei Normaldruck und 98°C Kopftemperatur eingespeist. Als Katalysator wurde Schwefelsäure eingesetzt. Trioxan wurde in der Kolonne zum Kopf hin auf etwa 35 Gew.-% aufkonzentriert. In einer nachgeschalteten Destillationskolonne wurden Leichtsieder abgetrennt. Der abgetrennte Leichtsiederstrom bestand aus 56 Gew.-% Methylformiat, 26 Gew.-% Methylal, 9 Gew.-% Methanol, 6 Gew.-% DOE, sowie aus geringen Mengen Wasser und Formaldehyd. Das entstandene Gemisch aus Trioxan, Formaldehyd und Wasser (35,5 Gew.-% Trioxan, 26,7 Gew.-% Formaldehyd, 36 Gew.-% Wasser, Rest: Nebenkomponenten, hauptsächlich Methanol) wurde in einem Rohrkristallisator mit Wendelrührer (Laborversion eines Kühlscheibenkristallisators, Volumen: 5 1) ausgehend von 38°C diskontinuierlich kristallisiert (Abkühlgeschwindigkeit: 3 K/h, Endtemperatur: 21°C). Das Kristallisat wurde auf einer Siebbecher-Zentrifuge bei 2000 U/min innerhalb von 3 min von der Mutterlauge abgetrennt. Das ungewaschene Kristallisat wies folgende Zusammensetzung auf: 97,3 Gew.-% Trioxan, 1,2 Gew.-% Formaldehyd, 1,4 Gew.-% Wasser, 0,1.Gew.-% Schwersieder. Das kristallisierte Trioxan wurde in einem isolierten Behälter unter Stickstoff-Atmosphäre bei Normaldruck aufgeschmolzen und mit einer Temperatur von 95°C in eine Destillationskolonne überführt, die bei Normaldruck betrieben wurde. Trioxan wurde als dampfförmiger.Seitenabzug gewonnen und anschließend kondensiert. Die Reinheit des Trioxans nach der Destillation lag bei >99,5 Gew.-% sowie < 50 ppm Wasser, < 5 ppm Ameisensäure, < 100 ppm DOE, 160 ppm TOE.

### Beispiel 2:

### Diskontinuierliche Kristallisation, Waschen mit Wasser (Endtemperatur: 10°C)

Ein Gemisch aus Trioxan, Formaldehyd und Wasser (35,5 Gew.-% Trioxan, 26,7 Gew.-% Formaldehyd, 36 Gew.-% Wasser) wurde analog zu dem Vorgehen in Beispiel 1 kristallisiert. Die Kristallisations-Endtemperatur betrug 10°C. Das Kristallisat wurde im Verhältnis 1:1 (volumetrisch) mit Wasser gewaschen und wies nach dem Waschen eine Reinheit von 95,3 Gew-% auf (0,2 Gew.-% Formaldehyd, 4,4 Gew.-% Wasser, 0,1 Gew.-% Schwersieder). Die Ausbeute an Trioxan lag bei 73 %.

### Beispiel 3:

### Kontinuierliche Kristallisation bei 10°C, ohne Waschschritt

Ein Gemisch aus Trioxan, Formaldehyd und Wasser (36,7 Gew.-% Trioxan, 26,4 Gew.-% Formaldehyd, 35,4 Gew.-% Wasser) wurde in einem Rohrkristallisator mit Wendelrührer kontinuierlich bei 10°C mit einer Verweilzeit von 1 h kristallisiert. Das Kristallisat wurde auf einer Siebbecher-Zentrifuge bei 2000 U/min innerhalb von 30 s von der Mutterlauge abgetrennt. Das ungewaschene Kristallisat wies folgende Zusammensetzung auf: 94,5 Gew.-% Trioxan, 2,7 Gew.-% Formaldehyd, 5 Gew.-% Wasser.

### Beispiel 4:

### Kontinuierliche Kristallisation bei 10°C, Waschen mit Wasser

Ein Gemisch aus Trioxan, Formaldehyd und Wasser (36,7 Gew.-% Trioxan, 26,4 Gew.-% Formaldehyd, 35,4 Gew.-% Wasser) wurde analog zu Beispiel 3 kristallisiert, wobei jedoch eine Verweilzeit von 2 h eingestellt wurde. Das Kristallisat wurde nach der Mutterlaugenabtrennung im Verhältnis (volumetrisch) 1:1 mit Wasser gewaschen (Waschschritt: 30 s, Trockenschleudern: 180 s, jeweils bei 2000 U min⁻¹). Das gewaschene Kristallisat hat eine Reinheit von 92,9 Gew.-% (0,1 Gew.-% Formaldehyd, 7 Gew.-% Wasser). Der Formaldehydgehalt im Kristallisat wurde durch die Verdrängungswäsche mit Wasser deutlich reduziert. Der höhere Wassergehalt war auf anhaftendes Waschwasser zurückzuführen. Die Trioxan-Ausbeute betrug 79 %.

### Beispiel 5:

### Kontinuierliche Kristallisation bei 10°C, Waschen mit Wasser und Methanol

Ein Gemisch aus Trioxan, Formaldehyd und Wasser (36,7 Gew.-% Trioxan, 26,4 Gew.-% Formaldehyd, 35,4 Gew.-% Wasser) wurde analog zu Beispiel 4 kristallisiert. Das Kristallisat wurde nach der Mutterlaugenabtrennung zweimal im Verhältnis 1:1 (volumetrisch) mit Wasser und einmal mit Methanol gewaschen (je Waschschritt: 30 s, Trockenschleudern: 180 s, jeweils bei 2000 U min⁻¹). Das Kristallisat war anschließend nahezu formaldehyd- und wasserfrei und wies folgende Zusammensetzung auf: 99 Gew.-% Trioxan, 0,02 Gew.-% Formaldehyd, 0,3 Gew.-% Wasser. Durch die stärkere Auflösung von Kristallisat beim mehrfachen Waschen sank die Trioxan-Ausbeute auf 53 %.

## Patentansprüche

1. Verfahren zur Herstellung von Trioxan durch Trimerisierung von Formaldehyd in wässriger saurer Lösung und anschließender Abtrennung von Trioxan aus einem im wesentlichen aus Trioxan, Wasser und Formaldehyd bestehenden Gemisch (Trioxan-Rohprodukt), **dadurch gekennzeichnet, dass** man
a) das Trioxan-Rohprodukt aus der Trimerisierung destilliert,
b) das Trioxan auskristallisiert und
c) von der Mutterlauge abtrennt sowie
d) anschließend aufschmilzt und
e) weitere Nebenprodukte abdestilliert.

2. Verfahren nach Anspruch-1, **dadurch gekennzeichnet, dass** man das Trioxan aus der Stufe e) austrägt und in die Polymerisation überführt.

3. Verfahren nach den Ansprüchen 1 oder 2, **dadurch gekennzeichnet, dass** man die Mutterlauge der Stufe c) zur Rückgewinnung von Formaldehyd und Trioxan destilliert.

4. Verfahren nach Anspruch 3, **dadurch gekennzeichnet, dass** man den überwiegend formaldehydhaltigen Strom aus der Destillation der Mutterlauge in die Trimerisierung zurückführt und den überwiegend trioxanhaltigen Strom in die Stufe a) zurückführt.

5. Verfahren nach den Ansprüchen 1 bis 4, **dadurch gekennzeichnet, dass** man Stufe b) als Suspensionskristallisation ausführt.

6. Verfahren nach den Ansprüchen 1 bis 5, **dadurch gekennzeichnet, dass** man Stufe b) des Verfahrens einstufig durchführt.

7. Verfahren nach den Ansprüchen 1 bis 6, **dadurch gekennzeichnet, dass** man eine Endtemperatur von ≤ 20°C bei Stufe b) des Verfahrens einstellt.

8. Verfahren nach den Ansprüchen 1 bis 7, **dadurch gekennzeichnet, dass** man das kristallisierte Trioxan nach der Abtrennung der Mutterlauge (Stufe c) wäscht und anschließend aufschmilzt (Stufe d).

9. Verfahren nach den Ansprüchen 1 bis 7, **dadurch gekennzeichnet, dass** man das kristallisierte Trioxan mit Wasser und/oder Alkoholen wäscht.

10. Verfahren nach den Ansprüchen 7 oder 8, **dadurch gekennzeichnet, daß** man das kristallisierte Trioxan bei Temperaturen von ≤ 20°C wäscht.

## Claims

1. A process for preparing trioxane by trimerizing formaldehyde in aqueous acidic solution and subsequently removing trioxane from a mixture substantially comprising trioxane, water and formaldehyde (crude trioxane product), which comprises
a) distilling the crude trioxane product from the trimerization,
b) crystallizing the trioxane out and
c) removing it from the mother liquor and
d) subsequently melting it and
e) distilling off further by-products.

2. The process according to claim 1, wherein the trioxane is discharged from stage e) and is transferred to a polymerization stage.

3. The process according to claim 1 or 2, wherein the mother liquor of stage c) is distilled to recover formaldehyde and trioxane.

4. The process according to claim 3, wherein the predominantly formaldehydic stream from the distillation of the mother liquor is recycled into the trimerization and the predominantly trioxanic stream is recycled into stage a).

5. The process according to claims 1 to 4, wherein stage b) is carried out as a suspension crystallization.

6. The process according to claims 1 to 5, wherein stage b) of the process is carried out in one stage.

7. The process according to claims 1 to 6, wherein an end temperature of ≤ 20°C is set in stage b) of the process.

8. The process according to claims 1 to 7, wherein the crystallized trioxane after the removal of the mother liquor (stage c) is washed and subsequently melted (stage d).

9. The process according to claims 1 to 7, wherein the crystallized trioxane is washed with water and/or alcohols.

10. The process according to claim 7 or 8, wherein the crystallized trioxane is washed at temperatures of ≤ 20°C.

## Revendications

1. Procédé de préparation de trioxanne par trimérisation de formaldéhyde dans une solution acide aqueuse et ensuite isolement du trioxanne à partir d'un mélange constitué essentiellement de trioxanne, d'eau et de formaldéhyde (produit brut de trioxanne), **caractérisé en ce que**
a) on distille le produit brut de trioxanne issu de la trimérisation,
b) on sépare le trioxanne par cristallisation, et
c) on l'isole de la lessive mère, ainsi que
d) on le fait ensuite fondre et
e) on sépare d'autres produits secondaires par distillation.

2. Procédé suivant la revendication 1, **caractérisé en ce qu'**on décharge le trioxanne de l'étape e) et on le fait passer dans la polymérisation.

3. Procédé suivant les revendications 1 ou 2, **caractérisé en ce qu'**on distille la lessive mère de l'étape c) en vue d'une récupération de formaldéhyde et de trioxanne.

4. Procédé suivant la revendication 3, **caractérisé en ce qu'**on recycle dans la trimérisation le courant contenant de manière prépondérante du formaldéhyde qui est issu de la distillation de la lessive mère et on recycle dans l'étape a) le courant contenant de manière prépondérante du trioxanne.

5. Procédé suivant les revendications 1 à 4, **caractérisé en ce qu'**on réalise l'étape b) sous la forme d'une cristallisation en suspension.

6. Procédé suivant les revendications 1 à 5, **caractérisé en ce qu'**on effectue l'étape b) du procédé en une étape.

7. Procédé suivant les revendications 1 à 6, **caractérisé en ce qu'**on ajuste une température finale de ≤ 20°C à l'étape b) du procédé.

8. Procédé suivant les revendications 1 à 7, **caractérisé en ce qu'**on lave le trioxanne cristallisé après l'isolement de la lessive mère (étape c) et ensuite on le fait fondre (étape d).

9. Procédé suivant les revendications 1 à 7, **caractérisé en ce qu'**on lave le trioxanne cristallisé avec de l'eau et/ou des alcools.

10. Procédé suivant les revendications 7 ou 8, **caractérisé en ce qu'**on lave le trioxanne cristallisé à des températures de < 20°C.
